# EUROPEAN PATENT APPLICATION

(11) **EP 4 491 634 A1**
(43) Date of publication of application: **15.01.2025**
(21) Application number: 24188265.3
(22) Date of filing: 12.07.2024
(51) Int. Cl.: C07K 16/28, C07K 16/30, A61P 35/00, A61P 35/04, A61K 39/395, G01N 33/574, G01N 33/577

(54) **ANTI-TROP2 ANTIBODIES FOR USE IN TREATING IMMUNOSUPPRESSED SUBJECTS**

(30) Priority: 14.07.2023 EP 23185456
(71) Applicant: ADRIATX S.r.l., 66034 Lanciano (IT)
(72) Inventor: ALBERTI, Saverio, 66034 LANCIANO (IT)
(74) Representative: Rigamonti, Dorotea

(57) **Abstract**

It forms an object of the present invention a composition comprising the 4F6 anti-Trop-2 antibody and/or its conjugates for use in the treatment of a Trop-2-expressing tumors in a subject, wherein said subject is immunosuppressed by anticancer treatments, such as chemotherapy or radiotherapy.

## Description

### Background of the invention

Trop-2 (AC: P09758), also known as EGP-1, T16, MR23, MR54, is a type-I transmembrane protein, encoded by the tumor-associated calcium signal transducer 2 (*TACSTD2*/*M1S1*/*GA733-1*) gene [Fornaro, 1995], a retrotransposon of the *TROP1*/*TACSTD1*/*EPCAM* gene [Linnenbach, 1993]. The extracellular domain of human Trop-2 (ECD, residues 27-274) encompasses a cysteine-rich N-terminal region, which hosts a GA733 type 1 motif (residues 27-69), and a thyroglobulin type-1 domain (residues 70-148), followed by a C-terminal domain devoid of cysteines (residues 149-274) [Fornaro, 1995]. A single transmembrane helix connects the ECD to a 26-amino acid (AA) intracellular tail, which contains a HIKE motif [Ciccarelli, 2000], and two PKC phosphorylation sites, at Ser303 [Basu, 1995] and Ser322 [Mori, 2019], and drives downstream Ca²⁺, PKC and AKT signaling [Guerra, 2022; Guerra, 2016].

Trop-2 induces tumor and cancer stem cell growth [Stoyanova, 2012; Trerotola, 2013a, 2013b]. Up-regulation of Trop-2 has been associated to poor prognosis of pancreatic, gastric, ovarian, lung and colorectal cancers [Relli, 2018; Guerra, 2021], indicating a key role of this molecule in tumor progression [Trerotola, 2013a, 2013b].

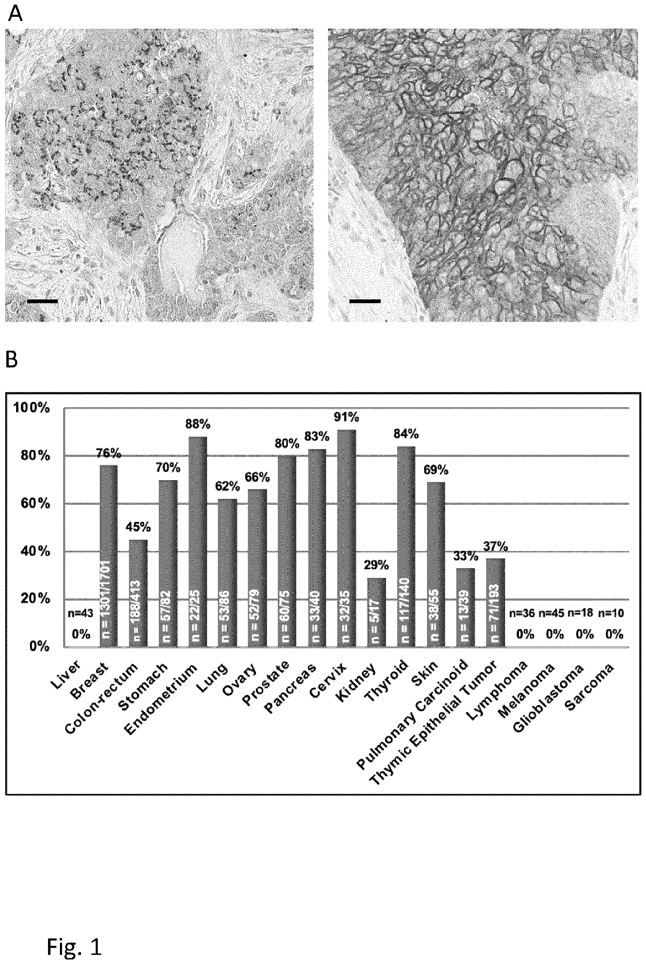

We have shown that Trop-2 is expressed by the majority of tumors in human [Trerotola, 2013a]. DNA microarray, EST, SAGE, Northern blot e RT-PCR analyses showed *TROP2* mRNA expression in ovarian, NSCLC, prostate, breast and colon cancer cell lines [Guerra, 2021] and in stomach, colon, breast, endometrium, kidney, lung and ovary human tumors [Trerotola, 2021; Guerra, 2021]. *TROP2* mRNA expression was also detected in normal human breast tissue, in lung, uterus, pancreas, prostate, salivary glands, respiratory ducts, thymus, kidney and placenta [Trerotola, 2013a]. An immunohistochemical analysis of Trop-2 protein expression was performed on over 3,000 human tumors, showing Trop-2 protein is highly expressed in the vast majority of malignant carcinomas (ranging between 64% and 90%) **(****Figure 1B****).**

### Trop-2 in growth control

A direct role for Trop-2 in tumor development was demonstrated both *in vitro* and *in vivo. De novo* Trop-2 expression in the murine thymic immortalized cell line MTE 4-14 and human ovarian tumor Igrov-1 or Trop-2 overexpression in the human colon cancer cell line KM12SM resulted in growth stimulation both in fully transformed and in simply immortalized cells [Trerotola, 2013a], indicating that Trop-2 expression is both necessary and sufficient to stimulate the growth of tumor cells.

Similar results were obtained in experimental tumors (293 or L tumor cells injected subcutaneously in nude mice) [Trerotola, 2013a]. Trop-2 expression was shown to induce an increase in mitotic activity, nuclear pleomorphism and the appearance of multinucleate giant cells. Tumor development was proportional to Trop-2 expression levels.

Deletion of the Trop-2 cytoplasmic tail abolished Trop-2-depended growth stimulation, thus showing that this region plays a key role in Trop-2 signaling [Guerra, 2013; Guerra, 2022]. The tail contains a serine residue (S303) that is phosphorylated by PKC [Basu, 1995]. S303 mutagenesis abolished growth stimulation, indicating that Trop-2 stimulatory activity is phosphorylation-dependent [Guerra, 2013; Guerra, 2022].

Proteomic and phosphoproteomic analyses on antibody arrays identified downstream molecules involved in Trop-2 signaling [Guerra, 2013; Guerra, 2022]. Trop-2-induced expression modifications were shown for trans-membrane tyrosine-kinase receptors (PDGFR, Met, Ret, VEGFR), soluble tyrosine-kinases, serine/threonine-kinases, phosphatases, cell-cycle and apoptosis regulatory molecules. Western blot analyses validated microarray analyses for a reference set of molecules and phosphorylation sites. The results obtained indicated Trop-2-dependent activation of PKCα, FAK and Raf-1, involvement of the PTEN-Akt-GSK3β-S6K pathway, modulation of ERK, JNK and p38 MAPK, induction of NF-B and modulation of apoptotic factors, p53 and Rb. The modulation of the key players of this model (e.g. ERK, Cyclin D1, NFkB) was shown to be dependent on the presence of an intact Trop-2 cytoplasmic tail.

### Trop-2 in metastatic spreading

Metastatic spreading is the main cause of death in many cancers in human. Common tumors such as colon, breast and lung cancers have in many cases generated metastases at the time of surgery. Metastatic tumors are often resistant to most of the currently available therapies [De Vita, 2001]. Therefore, a better knowledge of the molecular mechanisms at the basis of tumor spreading could play a critical role for a better treatment of advanced neoplastic disease. The identification of new markers of tumor aggressiveness and metastatic potential can contribute to the identification of the most aggressive cases at an early stage and provide new targets for novel therapies.

TROP2 is the only gene that is overexpressed in metastatic cells in different experimental systems. A large-scale analysis of Trop-2 expression in human primary cancers and their corresponding metastases revealed that Trop-2 is overexpressed in the metastases from colon, stomach, breast and ovary tumors [Guerra, 2021; Trerotola, 2021]. This was confirmed by Northern and Western blot and immunohistochemistry analyses.

A causative role for Trop-2 in metastatic spreading was demonstrated, using the HCT116 U.5.5 colon cancer cells and by transfection of the KM12SM colon tumor cell line with wild-type or mutagenized Trop-2. Transfected cells were then injected in the spleen of nude mice and metastatic potential was assessed. Trop-2 expression was shown to induce an increase in the metastatic spreading to the liver (90% of cases). Trop-2 expression also modified growth pattern, apoptosis induction, cell morphology, cell-replication rate and metastasis dimensions [Guerra, 2021; Trerotola, 2021].

The high frequency and expression levels of Trop-2 in human tumors and corresponding metastases have made this molecule an attractive target for adoptive immunotherapy. Most anti-Trop-2 monoclonal antibodies (mAb), amongst which MOv16 [Alberti, 1992], cAR47A6.4.2 [Truong, 2007], 162-46.2 [Alberti, 1992], Pr1E11 [Ikeda, 2015], AbT16 [Trerotola, 2021], bind one and the same immunodominant epitope [Alberti, 1992], in the D146-L274 region [Ikeda, 2015].

Limited efficacy versus difficult-to-manage toxicity severely affects current anti-Trop-2-targeted antibody therapy. In this context, a major stumbling block for next-generation therapeutic strategies remains the broad expression of Trop-2 in normal tissues [Trerotola, 2010]. We discovered that activation of Trop-2 for driving tumor progression requires proteolytic activation by ADAM 10 in cancer cells [Trerotola, 2021; Guerra, 2021]. Importantly, Trop-2 cleavage by ADAM10 was found not to occur in normal tissues [Trerotola, 2021; Guerra, 2021].

This provided the potential for a cancer-only Trop-2-targeting therapeutic strategy. A structure/function-guided analysis was conducted, to identify the cleavage-activated, signal-triggering region of Trop-2. Accessibility-directed activation-site recognition was then carried out, in order to target cancer-exposed sites, to exploit this unique cancer vulnerability in patients [Kim, 2022; Alberti, 2022]. Indeed, it is known how important it is that anti-tumor monoclonal antibodies (i.e., targeted against Her2/neu) are directed against specific portions of the target molecule, which play a fundamental role in determining the antitumor therapeutic efficacy [Johnson, 2006].

This also includes the possibility that antibody binding exerts direct functional inhibition of the antigen pro-growth, pro-metastatic signaling, resulting in tumor growth inhibition independent from the recruitment of cells of the immune system, namely T and B lymphocytes. This is especially important since most anticancer immunotherapy regimens are administered to patients as combinations with antiblastic chemotherapy, among them paclitaxel or anthracycline and cyclophosphamide. These can cause immunosuppression either directly, by killing anticancer T and B lymphocytes, or indirectly, by inducing immunosuppressive cell types or soluble circulating factors [Cersosimo, 2003]. Tumors themselves cause immunosuppression, and this has led to the use of antibodies against immunologic checkpoints to counteract it [Brody, 2011]. Distinct chemotherapy regimens can be identified that differentially modulate response to immune checkpoint inhibitors [Voorwerk, 2019].

Chemotherapy-induced damages can induce metastasis. Repair responses to chemotherapy are exploited by tumor cells from tumor cell local invasion, intravasation, hematogenous dissemination and extravasation with effective colonization at the metastatic site [Karagiannis, 2018].

Experimental evidence demonstrates that cancer therapy can induce host-mediated local and systemic responses, which can support tumor progression. Treatment with doxorubicin, but not cisplatin, induces the expression of complement factors in lung fibroblasts and modulates an immunosuppressive metastatic niche that supports lung metastasis [Monteran, 2022]. Additional mechanisms include therapy-induced immunological, angiogenic and pro-metastatic effects [Shaked, 2019]. In response to chemotherapy and radiation, cancer-associated fibroblasts and endothelial cells, respectively, generate elevated systemic levels of pro- inflammatory molecules such as interleukin-6 (IL-6) and IL-8. These secreted factors modulate the activity of myeloid cells including macrophages, that can act as suppressors of anti-tumor T and B lymphocytes, thus promoting tumor immune evasion [Shaked, 2019]. Treatment of healthy animal models with paclitaxel, cisplatin or gemcitabine chemotherapy leads to increased pulmonary metastasis following tumor cell inoculation, due to increased levels of circulating matrix metalloproteinase 9, SDF1 and granulocyte colony- stimulating factor (G-CSF). Chemotherapy-induced circulating growth factors and cytokines support tumor angiogenesis. Oxaliplatin induces an upregulation of circulating VEGFA. In addition, paclitaxel results in increased circulating levels of SDF1, G-CSF and VEGFC, increasing angiogenesis and tumor growth. Radiotherapy has also been reported to induce a pro-angiogenic shift [Shaked, 2019].

In parallel settings, the function of T and B lymphocytes was shown to require topoisomerase I activity, because of lymphocyte proliferation after encounter with the target antigen and activation of immune responses. Therefore, chemotherapeutic drugs that inhibit topoisomerase activity may alter systemic innate immune cell function and contribute to a pro- tumorigenic activity [Shaked, 2019]. In addition, systemic release of extracellular vesicles from B cells in response to chemotherapy resulted in impaired CD8+ T cell function, therefore contributing to immunosuppressive effects [Shaked, 2019]. ATP released from tumor cells in response to oxaliplatin, doxorubicin or cyclophosphamide chemotherapy is converted into adenosine and contributes to CD8+ T cell dysfunction [Shaked, 2019]. In response to 5-fluorouracil or gemcitabine chemotherapy, myeloid suppressor cells contribute to a pro-inflammatory local and systemic response that helps tumor progression [Shaked, 2019].

All these immunosuppressive mechanisms can severely affect cells that mediate antibody-dependent cell-mediated cytotoxicity (ADCC), such as NK cells and anti-tumor cytotoxic T lymphocytes and may result in resistance to antibody-based anticancer therapy.

Therefore, there was the precise need to generate new, homogeneous and high affinity anti-Trop-2 monoclonal antibodies, in particular directed against specific functional regions of the molecule, that, on top of ADCC, can also exert a direct inhibition of the tumorigenic role of Trop-2, for an efficient utilization in biomedical applications.

### Description

New anti-Trop-2 monoclonal antibodies were obtained with the aim of optimizing their recognition of specific functional regions of the target molecule. To achieve this, strategies were developed for the generation of monoclonal antibodies by immunization with recombinant Trop-2 proteins produced in baculovirus and mammalian cells, as they were expected to maintain the structure, folding, processing and glycosylation characteristics of native molecules. This led to select anti-Trop-2 hybridomas by ELISA tests. These were performed on recombinant Trop-2 proteins (both full-length or portions of the protein), to optimize reactivity with native Trop-2 proteins with correct folding and glycosylation. A second step of selection of anti-Trop-2 hybridomas was performed by cytofluorimetry, for reactivity on TROP2-transfected L cells, using as negative controls vector-alone control transfectants.

We discovered that Trop-2 undergoes proteolytic activation by ADAM 10 in cancer cells [Trerotola, 2021; Guerra, 2021]. This exposes a previously inaccessible protein groove, flanked by two glycosylation sites. We designed a recognition strategy of such targets, for exploiting selective cancer vulnerability in patients. Our strategy design was informed by structure-function analysis of the Trop-2 ECD [Trerotola, 2021; Pavšič, 2021]. This led to recognize that the N-terminal small subunit, which is severed from Trop-2 by ADAM10 cleavage at R87-T88 [Trerotola, 2021], masks a groove of the Trop-2 ECD and makes it largely inaccessible in the wt/uncleaved conformation. After ADAM10-mediated cleavage, the N-terminal small subunit only remains bound to the core Trop-2 structure via the Cys73-Cys108 disulfide bridge, and a profound rearrangement of the Trop-2 structure takes place, enabling the targeting of the groove thus uncovered.

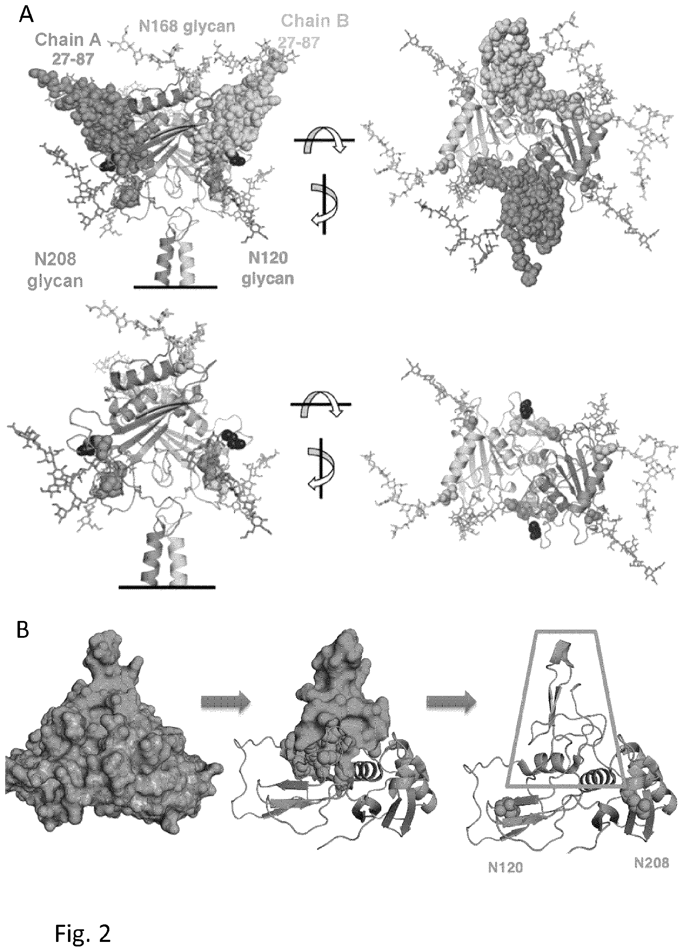

Through selective immunization and screening strategies we succeeded in generating a mAb family, that recognizes this region in an ADAM10 cleavage-dependent manner. The requirement for glycosylation at flanking sites supported the effective recognition of the cleavage-exposed target groove in Trop-2 **(****Figure 2****).**

There was the precise need to find high affinity anti-Trop-2 monoclonal antibodies, directed against specific functional regions of the molecule, that, on top of ADCC, can also exert a direct inhibition of the growth of cancer cells. This novel therapeutic strategy work also in patients that are immunosuppressed by chemotherapy, as it does not need interaction with functional immune cells, such as cytotoxic T lymphocytes, B lymphocytes and NK cells.

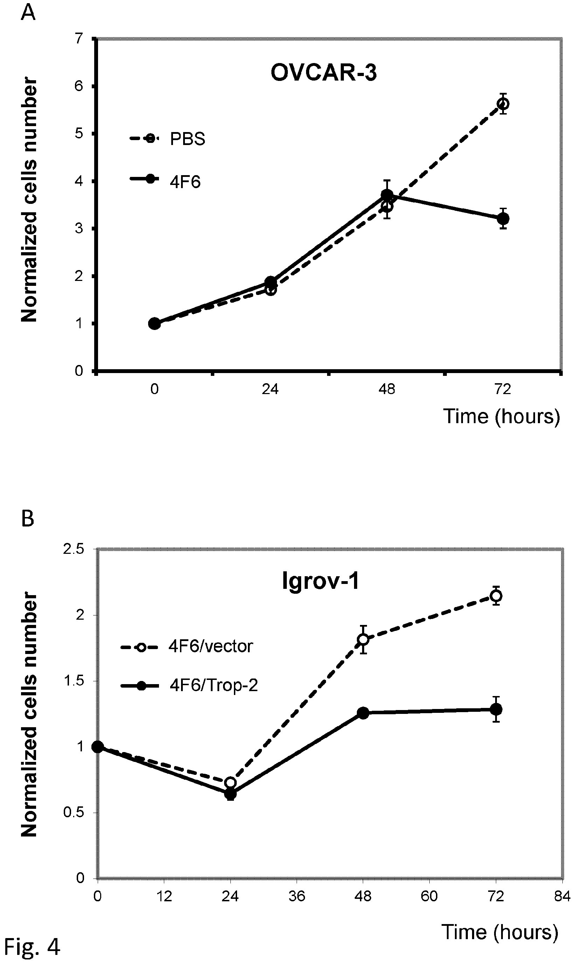

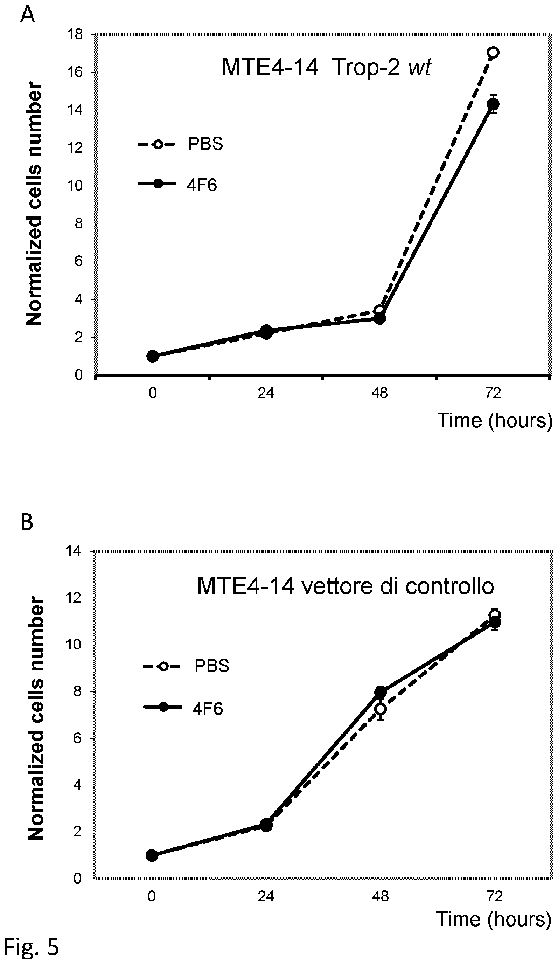

WO2010089782 describes the 4F6 monoclonal antibody, as specifically recognizing the activated Trop-2 in cancer cells. A direct inhibition of the growth of cancer cells by the 4F6 anti-Trop-2 monoclonal antibody was thus measured *in vitro,* in the absence of cells of the immune system. To avoid complement-dependent cytotoxicity (CDC) we used heat-inactivated serum products in all growth media. Heat inactivation at 56°C for 30 min destroys complement activity without affecting the serum growth-promoting ability. Surprisingly, we observed for the first time that the 4F6 antibody was able to inhibit cancer cell growth *in vitro* in the absence of ADCC and CDC **(****Figure 4**, 5). Growth inhibition was specific for Trop-2 expressing cells, since control vector-only transfectants were not affected **(****Figure 4**, 5).

These results indicated the capacity of 4F6 of being utilized for effective anticancer therapy in patients even in the presence of cancer- and chemotherapy-related immunosuppressive mechanisms.

### Brief description of the drawings

**Figure 1****:** Trop-2 expression in human tumors. (A) Trop-2 protein expression in cancer. IHC analysis of Trop-2 protein expression in human breast cancer samples. (left) Staining was performed with anti-Trop-2 murine mAb. Reactivity is prominent against intracellular Trop-2 deposits [Ambrogi, 2014]. (right) Staining was performed with anti-Trop-2 goat polyclonal antibody. Prevalent reactivity against the cancer cell-membrane Trop-2-activated form was observed [Ambrogi, 2014]. Bars = 30 µm. (B) Frequency of Trop-2 expression in human tumors. The tumor classes analyzed are listed on the X axis. Bar height is proportional to the frequency of expression of Trop-2 (the overall frequency is listed on top of the bar). Absolute numbers of cases analyzed are listed within each frequency bar.
**Figure 2****:** 3D structure of Trop-2. (A) Side and top (i.e. extracellular) views of the model of a human Trop-2 dimer [Pavšič, 2021 #36406], truncated in the middle of the transmembrane helices (horizontal black bar). As indicated by the arrow symbols, the two views are related by a 90° rotation around the horizontal direction. A black circle and a black rectangle surround the groove between the glycans at N120 and N208 that becomes more accessible upon ADAM10-cleavage and rearrangement of the N-terminal ADAM10-cleaved subunit. Trop-2 molecules in the dimer are in ribbon diagrams. Residues N120 and N208 are in spheres with the N-glycans at these sites in sticks. The N168 residue is in light gray spheres and its N-glycan is in light gray sticks. The two N-terminal ADAM 10-cleaved subunits (AA 27-87) are in dark gray spheres. The N33-linked glycans on this portion of the molecule are in dark gray sticks. The C73-C108 disulfide bridge that tethers the ADAM10-cleaved subunit to the rest of the protein is in black spheres. (top) Pre-cleavage Trop-2 dimer model. (*bottom*) Post-cleavage Trop-2 dimer model, with a possible novel orientation of the two N-terminal small subunits (AA 27-87) following ADAM-10 cleavage, preserving the C73-C108 disulphide bridge. (B) Full-length Trop-2 ECD. The N-terminal subunit is positioned at the top of the structure (gray spheres in the central diagram). Surface sphere models versus ribbon diagrams (on the right) is shown.

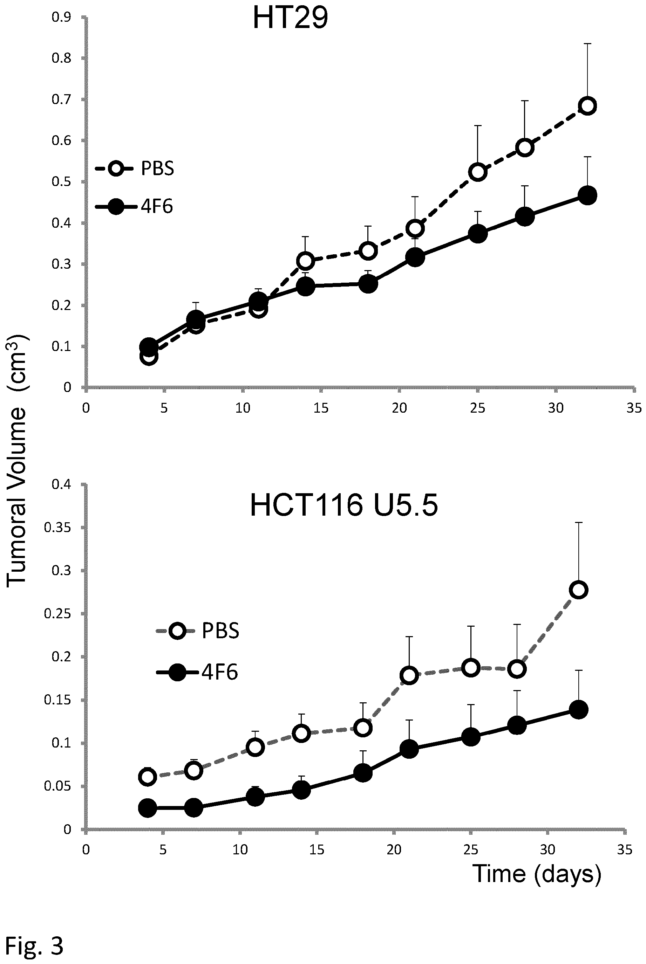

**Figure 3****:** Inhibition of tumor growth (human cancer cells) following treatment with the 4F6 anti-Trop-2 antibodies. Xenografts of non-metastatic HT29 colon cancer cells (top) or of metastatic HCT116 U5.5 colon cancer cells (bottom) treated with the 4F6 anti-Trop-2 mAb (solid black line), or with the PBS vehicle only (dotted line), starting from the time of injection. Bars represent the standard error of the mean of tumor volume measurements.
**Figure 4****:** Inhibition of ovarian cancer cell growth following treatment with the 4F6 anti-Trop-2 antibody. (A) Trop-2 expressing OVCAR-3 cancer cells were treated with the 4F6 antibody (solid black line) or with the PBS vehicle only (dotted line). (B) Igrov-1 cancer cells transfected withTrop-2 (solid black line) or with vector alone (control, dotted line) were treated with the 4F6 anti-Trop-2 antibody. Bars represent the standard error of the mean of cell number measurements.
**Figure 5****:** Inhibition of MTE4-14 transformed cell growth following treatment with the 4F6 anti-Trop-2 antibody. (A) MTE4-14 transfected with the wtTrop-2 were treated with the 4F6 antibody (solid black line) or with the PBS vehicle only (dotted line) as indicated. (B) MTE4-14 control vector-only transfectants were treated with the 4F6 antibody (solid black line) or with the PBS vehicle only (dotted line) as indicated. Bars represent the standard error of the mean of cell number measurements.

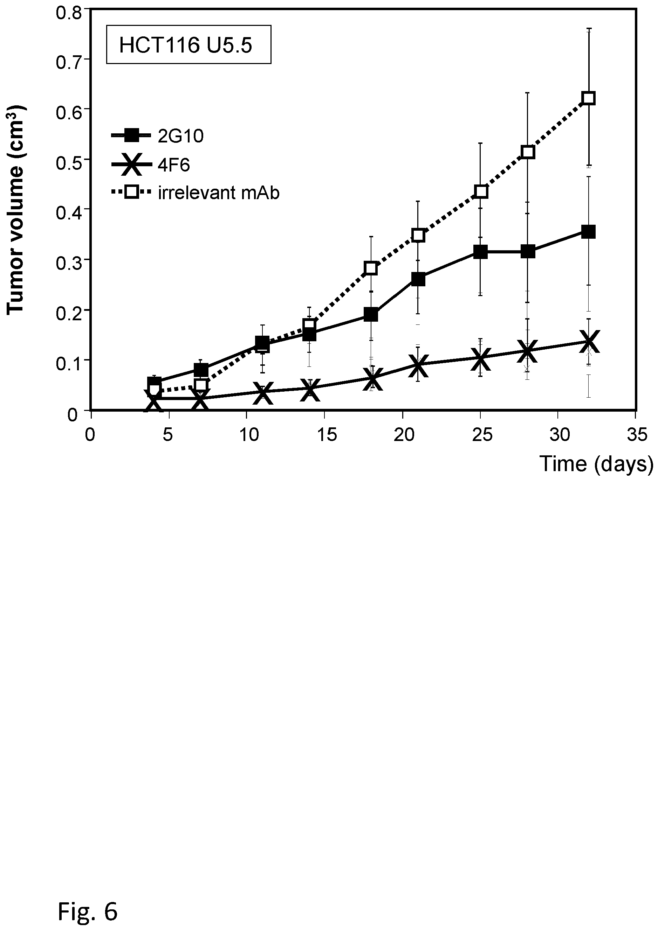

**Figure 6****:** Inhibition of the growth of the metastatic HCT116 U5.5 colon cancer cell line treated with 2G10 (comparative) or 4F6 (according to the invention) anti-Trop-2 monoclonal antibodies. Control tumors were treated with an antibody with irrelevant specificity (anti-dansyl: dotted line). Cells were treated in a prophylactic manner, i.e. by co-injection of tumor cells and antibodies. Antibody administration was performed i.p. once a week, for a total of 4 treatments. Bars represent the standard errors of the means of the measurements. In the treatment of a metastatic cell line 4F6 anti-Trop-2 antibody taught in the present invention is much more effective than the comparative 2G10 anti-Trop-2 antibody.

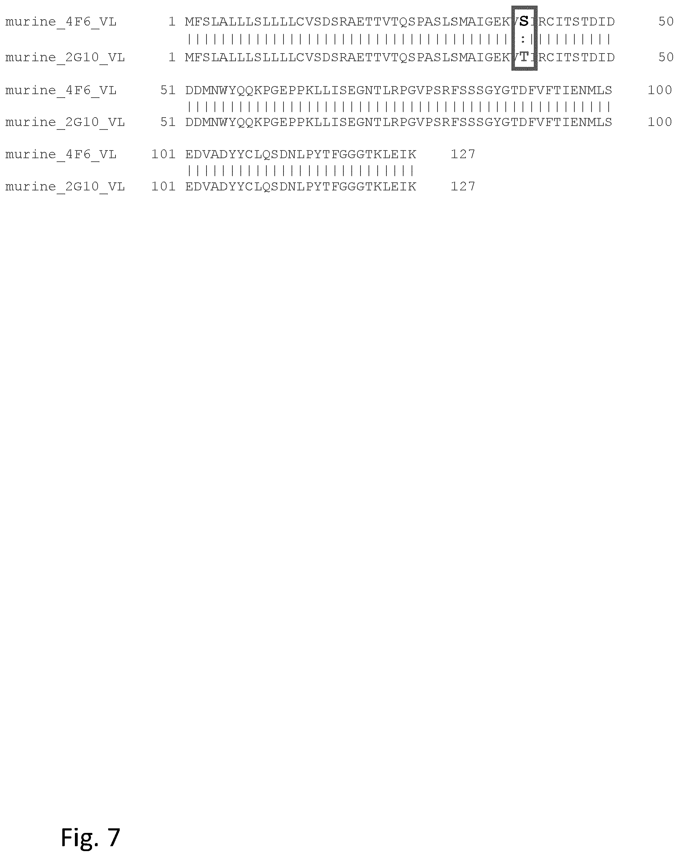

**Figure 7****:** Alignment of the VL sequence of the 2G10 (SEQ ID NO: 5) and 4F6 (SEQ ID NO: 4) antibodies.

### Detailed description

Object of the invention here presented is the use in anticancer therapy of the 4F6 anti-Trop-2 monoclonal antibody, and of its fully humanized or chimeric antibodies, where the murine constant region is substituted by human constant regions [Oi, 1983 #11830], or variants containing at least one of the complementarity determining regions (CDR) of the variable region of the corresponding light and/or heavy chains, possibly mutagenized to modify their affinity for the target, or antibody fragments, Fv, Fab, F(ab)2 fragments, single chain or multimeric 4F6 anti-Trop-2 antibody. In an embodiment, antibodies, fragments or antibody chimeras included in the invention here presented come from, or be engineered in, IgM, IgD, IgG, IgA, o IgE isotypes.

Another object of the invention here presented are the conjugated of the 4F6 antibody, wherein said antibody is conjugated with a biologically active partner, e.g. avidin or its derivatives, growth factors or toxins, cytokines, anti-tumor drugs and radioisotopes or other biologically active and/or useful to increase anti-tumor therapeutic effectiveness. As an example, said conjugates are selected in the group comprising:
(a) toxic payloads, optimized for anticancer potency;
(b) linkers, optimized to conjugate toxic payloads with high anticancer potency;
(c) beta particle emitting radioactive isotopes;
(d) alpha particle emitting radioactive isotopes for radioimmunotherapy;
(e) positron emitters for PET and PET-CT for imaging of Trop-2 expressing tumors and metastases.

Object of the invention here presented is the use in experimentation, diagnosis and therapy of the 4F6 anti-Trop-2 antibody conjugated to sequences, single residues or synthetic molecules (tags) for affinity chromatography purification. These tags can be used as detection molecules (e.g., radio isotopic or fluorescent tags) or enzymatic tags able to catalyze a visible substrate modification, both for diagnostic use in the lab and for imaging.

Object of the invention here presented are the modes of use in diagnostic applications, including but not limited to, optical, confocal, multiple photon and electronic microscopy, ELISA, Western blotting, immunoprecipitation, radioimmunological and similar techniques.

In another embodiment, it is an object of the present invention a composition comprising the 4F6 anti-Trop-2 antibody and/or its conjugates for the treatment of subjects having Trop-2-expressing tumors, wherein said subjects are immunosuppressed.

In another embodiment, it is an object of the present invention a method to treat a subject affected by a tumor expressing Trop-2, wherein said subject is immunosuppressed, said treatment being performed before or after tumor removal, with the 4F6 antibody or its conjugates, alone or in combination with other therapeutic interventions, such as other antibody-drug conjugates or other engineered molecules.

Objects of the invention here presented are the modes of use in therapy, in particular the administration modalities of the 4F6 anti-Trop-2 antibody and its conjugates, either systemic or locoregional, e.g. intraperitoneal, intra-hepatic artery or intratumor. An indicative but not exhaustive list of targets for therapies based on anti-Trop-2 antibodies includes endometrium, breast, head and neck, colon-rectum, stomach, lung, ovary, prostate, pancreas, cervix and bladder (urothelial) tumors.

Objects of the invention here presented are also the therapeutic compositions containing the 4F6 antibody or its conjugates. These can be provided as compositions or carrier agents or macro/micro or nanocontainers or nanostructures containing the antibody or its conjugates, together with a pharmaceutically acceptable delivery or dilution agent. The therapeutic composition can be used for the treatment of diseases in mammalian organisms such as humans. The 4F6 anti-Trop-2 antibody will be included in pharmaceutical compositions or diagnostic kits according to the normal practice in the field.

A further object of the invention here presented is the use of the 4F6 anti-Trop-2 antibody conjugates in the imaging of patients by means of technologies and procedures known in the art, through the labeling of the 4F6 anti-Trop-2 antibody with radioactive isotopes or fluorescent tracers, e.g., quantum dots or organic chromophores or enzymes which can be detected by chemiluminescence. The signal originated by labelled anti-Trop-2 antibodies is detectable by scanners or tomography instrumentation, according to the principles of currently used equipment such as TAC/PET.

A further object of the invention is the utilization of the radioactive isotope-bound 4F6 anti-Trop-2 antibody for anticancer therapy.

The 4F6 anti-Trop-2 monoclonal antibody of murine origin and its conjugates can be produced in reasonable amounts by researchers who are expert in the art.

The coding regions (open reading frame, ORF) and amino acid sequences corresponding to the VH and VL of 4F6 monoclonal antibody are listed below:
VH (SEQ ID NO: 1) 
CAGCTGCAGCAGTCTGGAGCTGAGGTGGTGAAGCGTGGGGCTTCAGTG
AAGCTGTCCTGCAAGACTTCTGGCTTCACCTTCAGCAGTAGCTATATAAG
TTGGTTGAAGCAGAAGCCTCGACAGAGTCTTGAGTGGATTGCATGGATT
ATGCTGGAACTGGTGGTACTAGCTATAATCAGAAGTTCACAGGCAAGGC
CCAACTGACTGTAGACACATCCTCCAGCACAGCCTACATGCAACTCAGC
AGCCTGACATCTGAGGACTCTGCCATCTATTACTGTGCAAGACATAACCC
TTGTTACTATGCTATGGATTACTGGGGTCAAGG
VH Amino acid sequence (SEQ ID NO: 2) 
DVQLEQFGAELVRPGTSVKMSCKAAGYTFTNYWIGWVKQRPGHGLEWIGD
IYPGGGYTNYNEKFKGKATLTADTSSSTAYMQLSSLTFEDFAIYYCARGTGG
GDYWGQG
VL (SEQ ID NO: 3): 
ATGTTCTCACTAGCTCTTCTCCTCAGTCTTCTTCTCCTCTGTGTCTCTGAT
TCTAGGGCAGAAACAACTGTGACCCAGTCTCCAGCATCCCTGTCCATGG
CTATAGGAGAAAAAGTCTCCATCAGATGCATAACCAGCACTGATATTGAT
 
GATGATATGAACTGGTACCAGCAGAAGCCAGGGGAACCTCCTAAGCTCC
TTATTTCAGAAGGCAATACTCTTCGTCCTGGAGTCCCATCCCGATTCTCC
AGCAGTGGCTATGGTACAGATTTTGTTTTTACAATTGAAAACATGCTCTCA
GAAGATGTTGCAGATTACTACTGTTTGCAAAGTGATAACTTGCCGTACAC
GTTCGGAGGGGGGACCAAGCTGGAAATAAAA
VL Amino acid sequence (SEQ ID NO: 4)

MFSLALLLSLLLLCVSDSRAETTVTQSPASLSMAIGEKVSIRCITSTDIDDDMN
WYQQKPGEPPKLLISEGNTLRPGVPSRFSSSGYGTDFVFTIENMLSEDVAD
YYCLQSDNLPYTFGGGTKLEIK

To note, the 4F6 VH and VL here presented are highly related to 2G10 VH and VL sequences, described in WO2010/089782. Figure 7 shows the alignment of the VL sequence of 2G10 (SEQ ID NO: 5) and 4F6 (SEQ ID NO: 4). There is a single point mutation Surprisingly, this difference among the two antibody is enough to increase the selective activity of 4F6 on metastatic cancer cells with respect to the activity observed when using 2G10.

Similar procedures are equally applicable relative to the antibody and its fragments, such as Fv, Fab and F(ab)₂ fragments and single-chain or multimeric anti-Trop-2 antibodies, including their engineered forms to obtain different isotypes (IgM, IgD, IgG, IgA, IgE) and the murine variable chain, as fully or partially humanized.

A nucleic acid molecule coding for anti-Trop-2 immunoglobulins can be generated by an expert in the art using established technologies, e.g., oligonucleotide synthesis or PCR amplification. The nucleic acid molecule, once synthetized, can be cloned into an expression vector, as known in the art.

Another method for the realization of the invention here presented consists of obtaining, for instance by transfection, cells which express 4F6, as included in the invention. The transfection methods are known, and transfection kits can be purchased from commercial sources (for instance, Invitrogen, Carlsbad, Ca; Stratagene, La Jolla, CA).

Another method for the realization of the invention here presented is by means of IHC or immunofluorescence for the detection or the diagnosis of neoplastic, pre-neoplastic and non-neoplastic diseases expressing target molecules for these antibodies, which can be exploited for their identification and quantification. Further diagnostic methodologies include Western blot and its derivatives, where proteins are fractionated by gel electrophoresis and then detected by specific antibodies.

### EXAMPLES

Following are examples that illustrate procedures for practicing the invention. These examples should not be construed as limiting.

### Example 1: Inhibition of growth of human cancer cells in vivo, following treatment with the 4F6 anti-Trop-2 antibody.

Colon cancer cell lines were injected subcutaneously (5-10×10⁶ cells) into 8-week-old female athymic CD1-Foxn1 nu/nu mice. Xenografts **(****Figure 3****)** were treated with the 4F6 anti-Trop-2 mAb, or with the PBS vehicle only, by weekly intraperitoneal administration of 800 µg/mouse of antibody in sterile PBS. Treatment was administered weekly, from the time of injection, for 4 weeks. The tumor longest/shortest diameters (D/d) were measured every 5-7 days, and tumor volumes were calculated as for an ellipsoid (D×d²/2) [Guerra, 2021]. ANOVA [Rossi, 2008] and t test implementing a post-hoc Bonferroni correction was used to comparatively assess tumor growth curves, considering the standard error of the mean of tumor volume measurements, using Sigma Stat (SPSS Science Software UK Ltd.) and GraphPad Prism (GraphPad Software Inc., La Jolla, Ca).

### Example 2: Efficacy of 4F6 treatment to inhibit the growth of cancer cells.

The OVCAR-3 and IGROV-1 ovarian cancer were seeded at 1.5-3×10³ cells/well in 96-well plates (five replica wells per data point). One µg/well of purified 4F6 in sterile PBS, or vehicle only as control, were added to the cell cultures. Cell numbers were quantified every 24 hours by staining with crystal violet [Orsulic, 2002]. The standard error of the mean of cell number measurements was determined **(****Figure 4****).**

### Example 3: Direct inhibition of the growth of human cancer cells, following treatment with 4F6, as specific for Trop-2-expressing cells.

Trop-2 expressing IGROV-1 and MTE4-14 cells transfected with Trop-2 were seeded at 1.5-3×10³ cells/well in 96-well plates (five replica wells per data point). Cells transfected with the empty vector were used as controls. One µg/well of purified 4F6 in sterile PBS or vehicle only as control (for MTE4-14 transfectants) were added to the cell cultures and cell numbers were quantified every 24 hours by staining with crystal violet [Orsulic, 2002]. The standard error of the mean of cell number measurements was determined **(****Figure 4B**,5).

### Example 4: Inhibition of the growth of metastatic human cancer cells, following treatment with 4F6, as specific for Trop-2-expressing cells.

Trop-2-expressing metastatic HCT116 U5.5 colon cancer cell line was injected in the flank of nude, i.e. immunosuppressed, mice, at 5×10⁶ cells per injection site. The mice were treated with 2G10 (comparative) or 4F6 (according to the invention) anti-Trop-2 monoclonal antibodies. Control tumors were treated with an antibody with irrelevant specificity (anti-dansyl: dotted line). Cells were treated in a prophylactic manner, i.e. by co-injection of tumor cells and antibodies. Antibody administration was performed i.p. once a week, for a total of 4 treatments. Bars represent the standard errors of the means of the measurements. In the treatment of a metastatic cell line 4F6 anti-Trop-2 antibody taught in the present invention is much more effective than the comparative 2G10 anti-Trop-2 antibody **(****Figure 6****).**

### Materials and Methods

**Flow Cytometry.** The binding of monoclonal antibodies to the surface of tumor and transfected cells was analyzed by flow cytometry on non-transformed and transformed cell lines. Staining and analysis were performed essentially as described [Alberti, 1994]. Monoclonal antibody amounts that did not stain the negative controls, i.e., untransfected or mock-transfected cells, while staining positive cells as close to saturation as possible, were used. In most cases, 0.3 µg MAb per 2 × 10⁵ cells in 200 µl proved adequate. To improve the detection of expressing cells, subtraction of cell autofluorescence and overcompensation in the red channel [Alberti, 1991] were performed. The latter procedure enabled us to distinguish true expressing cells from cells up taking shedded antigen. Forward scatter, side scatter, and propidium iodide gating were routinely used to eliminate dead cells and debris from the analysis.

**Trop-2 structure analysis.** A homology model for the ECD thyroglobulin-like repeat was constructed over the p41 splice variant of the major histocompatibility complex class II-associated invariant chain (PDB ID 1ICF, chain I) [Trerotola, 2021; Guncar, 1999]. The mature portion of human Trop-2, residues 27-323 (Uniprot P09758 (TACD2_HUMAN) was subsequently modelled as a cis-dimer analogous to the dimer in the crystal of human Trop-1/EpCAM (PDB ID 4MZV [Pavsic, 2014; Fornaro, 1995; Zanna, 2007]) and the dimer in the NMR-derived model of the trans-membrane region of the rat p75 protein (PDB ID 4MZV, residues 278-298 [Nadezhdin, 2016]). These protein models were used as templates. The cytoplasmatic domain of human Trop-2, portion 299-323, was inherited from the NMR structure of the C-terminus of Trop-2 (PDB ID 2MAE [Pavsic, 2015]).

The analysis of the human Trop-2 ECD was validated over the crystal structure in PDB ID 7PEE [Pavsic, 2015]. Structure files were from the Protein Data Bank (www.rcsb.org/). Swiss-PdbViewer (www.expasy.ch/swissmod/SWISS-MODEL.html), and PyMol (pymol.org/2/) were utilized for graphic rendering of the 3D structures. N-glycosylation was added to Asn residues N33, N120, N168 and N208 using the GlyProt server (www.glycosciences.de/modeling/glyprot/php/main.php). The list of residues at the interface between the N-terminal cleaved portion and the Trop-2 trans-membrane portion, and the area buried in this interface, were computed at the PISA server (www.ebi.ac.uk/pdbe/pisa/; www.ebi.ac.uk/pdbe/prot int/pistart.html).

**DNA transfection.** Cells were transfected with DNA [Alberti, 1994] in Lipofectamine 2000 or LTX (Invitrogen) following manufacturer instructions. Stable transfectants were selected in G-418-containing medium.

**ELISA.** ELISA assay plates were coated overnight at 4°C with 100 µl/well of 1 µg/ml recombinant human Trop-2-lgFc chimera protein (rhTROP-2; R&D, Cat #650-T2-100), in 0.2 M sodium carbonate buffer (pH 9.4). Well surfaces were blocked with 300 µl/well of blocking buffer (2% skim milk in PBS, 0.05% Tween-20), for 30 min at RT. The plates were washed 2x with wash buffer (PBS, 0.05% Tween-20). Purified antibodies or supernatants were added to the plates at serial 3-fold dilutions, starting from 5-10 µg/ml, 100 µl/well. All dilutions were performed in blocking buffer. Antibody-containing plates were incubated for 1 hr at RT. The plates were then washed 3x with wash buffer. Antibody binding was revealed with 100 µl/well of a 1:2000 dilution of goat anti-human kappa-HRP (Southern Biotech, Cat# 2060-05) in blocking buffer, incubated for 30 min at RT, and washed 4x with wash buffer. HRP activity was revealed with 100 µl/well ABTS substrate (AMRESCO, Solon, OH), activated with 20 µl 30% H₂O₂ per 10 ml ABTS solution. The reaction was stopped with 100 µl per well 2% oxalic acid. Absorbance was read at 405nm.

**Immunohistochemistry (IHC).** IHC of normal and neoplastic human tissues was performed as previously described [Ambrogi, 2014]. Briefly, specimens were fixed in phosphate-buffered formalin, pH 7.2 and embedded in paraffin. Five µm sections were mounted on silanized slides, deparaffinized, and rehydrated through graded alcohols to water. Endogenous peroxidase activity was eliminated by incubation with 3% H₂O₂ for 5 min. Antigen retrieval was performed by microwave treatment at 750 W for 10 min in 1 M urea buffer pH 8.0.

Sections were incubated for 30 min with the 162-46.2 anti-Trop-2 mAb (ATCC clone HB187) ascites at 1:500 dilution **(****Figure 1A****, left)** or the AF650 anti-Trop-2 goat pAb (R&D Systems) **(****Figure 1A****, right).** To control for non-specific reactivity, the specific primary antibodies were replaced with non-immune serum or with isotype-matched immunoglobulins (DAKO). Anti-mouse (K4001, EnVision kit; DAKO) and anti-goat (K0679, LSAB kit; DAKO) secondary pAb were used for signal amplification, as appropriate. Slides were washed in Tris-buffered saline-Tween 20 and incubated for 10 min in 3,3'-diaminobenzidine (DAKO). Counterstaining was performed with haematoxylin. Slides were mounted with Immunomount (Shandon).

Trop-2 expression was quantified as percentage of stained cells and as intensity of staining. An IHC score (H-score) was obtained (range: 0 to 12). Five classes of expression prevalence were categorized: 0 (0% of positive cells), 1 (<10 % of positive cells), 2 (10-50% of positive cells), 3 (50-80% of positive cells), 4 (>80% of positive cells). An intensity score classified average intensity of positive cells as 1 (weak staining), 2 (moderate staining) or 3 (strong staining). The positivity and intensity scores were then multiplied to obtain a final H-score, that ranked tumours for overall expression of Trop-2 **(****Figure 1B****). In vitro cell-growth assays.** Trop-2 expressing OVCAR-3 cells or IGROV-1 and MTE 4-14 cells transfected with the empty vector or with Trop-2 were seeded at 1.5-3×10³ cells/well in 96-well plates (five replica wells per data point). One µg of purified 4F6 in sterile PBS or vehicle only as control were added to the cell cultures and their impact on cell growth was measured. Cell numbers were quantified by staining with crystal violet [Orsulic, 2002 #4340]. **Experimental tumors.** Trop-2 expressing cancer cell lines were injected subcutaneously (5-10×10⁶ cells) into 8-week-old female athymic CD1-Foxn1 nu/nu mice (Charles River Laboratories, Calco, Lecco, Italy). The tumor longest/shortest diameters (D/d) were measured every 5-7 days. Tumor volumes were calculated as for an ellipsoid (D×d²/2) [Guerra, 2021]. Treatment with anti-Trop-2 mAb or vehicle only was performed by weekly intraperitoneal administration of 800 µg/mouse of antibody in sterile PBS, for 4 weeks starting from the day of the inoculation.

**Statistical analysis.** The Student t-test was used for comparison of mean protein levels in control and *TROP2* transfectants. Normality of distribution of assay values was verified (www.graphpad.com). Two-tailed Fisher exact tests were used to compare protein expression levels in normal *versus* tumor samples. EC₅₀ values were calculated from dose-response data fitted to a 4-parameter-logistic non-linear regression model. Spearman non-parametric correlation coefficients were computed for protein expression levels in human cancer samples. ANOVA [Rossi, 2008 #14010] and *t* test implementing a post-hoc Bonferroni correction were used to comparatively assess tumor growth curves. Data were analyzed using Sigma Stat (SPSS Science Software UK Ltd.) and GraphPad Prism (GraphPad Software Inc., La Jolla, Ca).

### Results

The therapeutic efficacy of the 4F6 anti-Trop-2 mAb was tested *in vivo* using human cancer cells injected in nude mice. Two colon cancer models were used, which endogenously express Trop-2: the HT29 colon cancer cells and the metastatic HCT116U5.5 clone, which was derived from the non-metastatic HCT116 colon cancer cell line by in vivo selection for acquisition of metastatic ability upon upregulation of Trop-2 expression [Guerra, 2021].

The HT29 **(****Figure 3****,** top) and HCT116 U5.5 **(****Figure 3****, bottom)** colon cancer cell lines were injected subcutaneously into the flank of nude mice, and tumor volumes were measured every 5-7 days [Guerra, 2021]. Xenograft-bearing mice were treated with 800 µg of endotoxin-free 4F6 mAb once a week for four weeks, starting at the time of cancer-cell injection. Mice in the control group received PBS vehicle only, which had been shown in previous experiments to be equivalent to an irrelevant isotype-matched mAb. ANOVA [Rossi, 2008] and *t* test implementing a post-hoc Bonferroni correction was used to comparatively assess tumor growth curves, taking into account the standard error of the mean of tumor volume measurements, for 35 days after tumor cell injection **(****Figure 3****).** These experiments showed statistically significant inhibition of tumor growth in mice injected with the 4F6 antibody, indicating the feasibility of use of 4F6 for anticancer therapeutic purposes.

Using the same procedures, the 4F6 anti-Trop-2 antibody was then compared with the 2G10 anti-Trop-2 antibody, for efficacy of inhibition of the growth of metastatic human cancer cells. The Trop-2-expressing metastatic HCT116 U5.5 colon cancer cell line was injected in the flank of nude, i.e. immunosuppressed, mice, at 5×10⁶ cells per injection site. The mice were treated with 2G10 (comparative) or 4F6 (according to the invention) anti-Trop-2 monoclonal antibodies. Control tumours were treated with an antibody with irrelevant specificity (anti-dansyl: dotted line). Cells were co-injected with the listed antibodies. Antibody administration of 800 µg of endotoxin-free/mouse was performed i.p. once a week, for a total of 4 treatments. ANOVA [Rossi, 2008] and *t* test implementing a post-hoc Bonferroni correction was used to comparatively assess tumor growth curves, taking into account the standard error of the mean of tumor volume measurements **(****Figure 6****).** These experiments showed statistically significant inhibition of tumor growth in mice injected with the 4F6 antibody. These experiments also showed that the 4F6 anti-Trop-2 antibody taught in the present invention is much more effective than the comparative 2G10 anti-Trop-2 antibody, in the treatment of a metastatic cell line, indicating the feasibility of use of 4F6 for anticancer therapeutic purposes in immunosuppressed patients at an advanced stage, i.e. with metastatic diffusion of the tumor.

The immunosuppression induced by chemotherapy hampers ADCC cytotoxicity and may result in resistance to antibody-based anticancer therapy. The function of T and B lymphocytes requires topoisomerase I activity and chemotherapeutic drugs that inhibit topoisomerase activity alter immune cell function [Shaked, 2019]. Corresponding inhibitory signals originate from ATP released from tumor cells in response to chemotherapy such as oxaliplatin, doxorubicin or cyclophosphamide [Shaked, 2019]. In response to 5-fluorouracil or gemcitabine chemotherapy, myeloid suppressor cells contribute to tumor progression [Shaked, 2019].

Therefore, there was the precise need to generate new, homogeneous and high affinity anti-Trop-2 monoclonal antibodies, directed against specific functional regions of the molecule, that, on top of ADCC, can also exert a direct inhibition of the tumorigenic role of Trop-2, for an efficient utilization in biomedical applications. Our findings demonstrate that this important property is possessed by 4F6, as described below.

As 4F6 can directly inhibit the growth of cancer cells, it was important to show that 4F6 can exert a direct inhibition of the tumorigenic role of Trop-2. Cell culture growth assays were thus set-up to show that the efficacy of 4F6 treatment was specific for the expressed Trop-2 target protein.

Trop-2- expressing IGROV-1 and MTE4-14 transfectants were seeded at 1.5-3×10³ cells/well in 96-well plates. Cells transfected with the empty vector were used as controls. One µg of purified 4F6 in sterile PBS, or vehicle only as control for MTE4-14 transfectants, were added to the cell cultures after attachment to the substrate. The impact of 4F6 treatment on cell growth was measured by cell number quantification with the crystal violet staining method [Orsulic, 2002 #4340] up to 72 hours after seeding. Specific inhibition of cell growth by a treatment with the 4F6 anti-Trop-2 antibody was obtained in Trop-2-expressing IGROV-1 ovarian cancer cells. On the other hand, none was seen for empty-vector mock transfectants **(****Figure 4B****).** Further specific inhibition of cell growth was also shown for Trop-2-expressing MTE4-14 cells following treatment with the 4F6 anti-Trop-2 antibody, as compared to empty-vector mock transfectants and vehicle-only treatment **(****Figure 5****).**

Hence, significant direct inhibition of cancer cell growth is exerted by the 4F6 anti-Trop-2 antibody, indicating the feasibility of tumor growth inhibition by 4F6 even in the absence of ADCC, for example in patient immunosuppressed by chemotherapy or radiotherapy.

Finally, the 4F6 antibody was tested on HCT116 U5.5 cells, in comparison to 2G10 antibody. Colon cancer cells were co-injected with the indicated antibodies (according to the invention, 4F6; comparative, 2G10, or control, anti-dansyl) subcutaneously into the flank of nude mice, and tumor volumes were measured every 5-7 days. The experiment showed statistically significant inhibition of tumor growth in mice co-injected with 4F6 antibody. The inhibition was inferior when injecting the 2G10 antibody **(****Figure 6****).** To note, the different activity of the two antibodies is observed on HCT116 U5.5 cell line, a metastatic clone of colon cancer. The 4F6 antibody is not more effective when tested on HT29 cells, non-metastatic tumor cells. These data confirm the advantageously selective effect of 4F6 antibody on activated Trop-2, wherein activated Trop-2 induces metastatization, and indicate the feasibility of use of 4F6 for anticancer therapeutic purposes in immunosuppressed patients at an advanced stage, and with metastatic diffusion of the tumor.

To note, the 4F6 VH and VL here presented are highly related to 2G10 VH and VL sequences, described in WO2010/089782. Surprisingly, the difference in sequence of the VL among the two antibodies **(****Figure 7****)** is enough to increase the selective activity and recognition of 4F6 for Trop-2 in metastatic cancer cells with respect to the activity observed when using 2G10.

### References:

Alberti, S., C. Bucci, M. Fornaro, A. Robotti and M. Stella (1991). "Immunofluorescence analysis in flow cytometry: better selection of antibody-labeled cells after fluorescence overcompensation in the red channel." J. Histochem. Cytochem. 39(5): 701-706.
Alberti, S., S. Miotti, M. Stella, C. E. Klein, M. Fornaro, S. Ménard and M. I. Colnaghi (1992). "Biochemical characterization of Trop-2, a cell surface molecule expressed by human carcinomas: formal proof that the monoclonal antibodies T16 and MOv-16 recognize Trop-2." Hybridoma 11: 539-535.
Alberti, S., M. Trerotola and E. Guerra (2022). "The Hu2G10 tumor-selective anti-Trop-2 monoclonal antibody targets the cleaved-activated Trop-2 and shows therapeutic efficacy against multiple human cancers." Cancer Res 82 (12_Supplement): 340.
Alberti, S., M. Nutini and L. A. Herzenberg (1994). "DNA methylation prevents the amplification of TROP1, a tumor associated cell surface antigen gene." Proc. Natl. Acad. Sci. USA 91: 5833-5837.
Ambrogi, F., M. Fornili, P. Boracchi, M. Trerotola, V. Relli, P. Simeone, R. La Sorda, R. Lattanzio, P. Querzoli, M. Pedriali, M. Piantelli, E. Biganzoli and S. Alberti (2014). "Trop-2 is a determinant of breast cancer survival." PLoS One 9(5): e96993.
Basu, A., D. M. Goldenberg and R. Stein (1995). "The epithelial/carcinoma antigen EGP-1, recognized by monoclonal antibody RS7-3G11, is phosphorylated on serine 303." Int J Cancer 62(4): 472-479.
Brody, J., H. Kohrt, A. Marabelle and R. Levy (2011). "Active and passive immunotherapy for lymphoma: proving principles and improving results." J Clin Oncol 29(14): 1864-1875.
Cersosimo, R. J. (2003). "Monoclonal antibodies in the treatment of cancer, Part 2." Am J Health Syst Pharm 60(16): 1631-1641.
Ciccarelli, F., A. Acciarito and S. Alberti (2000). "Large and diverse numbers of human diseases with HIKE mutations." Hum. Mol. Genet. 9: 1001-1007.
De Vita, V. T., S. Hellman and S. A. Rosenberg (2001). Cancer - Principles and Practice of Oncology. Philadelphia, Lippincott J. B. Co.
Fornaro, M., R. Dell'Arciprete, M. Stella, C. Bucci, M. Nutini, M. G. Capri and S. Alberti (1995). "Cloning of the gene encoding TROP-2, a cell-surface glycoprotein expressed by human carcinomas." Int. J. Cancer 62: 610-618.
Guerra, E., V. Relli, M. Ceci, R. Tripaldi, P. Simeone, A. L. Aloisi, L. Pantalone, R. La Sorda, R. Lattanzio, A. Sacchetti, K. Havas, S. Guarnieri, D. Vergara, I. Fournier, M. Salzet, N. Tinari, M. Piantelli, M. Trerotola and S. Alberti (2022). "Trop-2, Na+/K+ ATPase, CD9, PKCα, cofilin assemble a membrane signaling super-complex that drives colorectal cancer growth and invasion." Oncogene 41(12): 1795-1808.
Guerra, E., M. Trerotola, A. L. Aloisi, R. Tripaldi, G. Vacca, R. La Sorda, R. Lattanzio, M. Piantelli and S. Alberti (2013). "The Trop-2 signalling network in cancer growth." Oncogene 32: 1594-1600.
Guerra, E., M. Trerotola, V. Relli, R. Lattanzio, R. Tripaldi, G. Vacca, M. Ceci, K. Boujnah, V. Garbo, A. Moschella, R. Zappacosta, P. Simeone, R. de Lange, U. H. Weidle, M. T. Rotelli, A. Picciariello, R. Depalo, P. Querzoli, M. Pedriali, E. Bianchini, D. Angelucci, G. Pizzicannella, C. Di Loreto, M. Piantelli, L. Antolini, X.-F. Sun, D. F. Altomare and S. Alberti (2021). "Trop-2 induces ADAM10-mediated cleavage of E-cadherin and drives EMT-less metastasis in colon cancer." Neoplasia 23(9): 898-911.
Guerra, E., M. Trerotola, R. Tripaldi, A. L. Aloisi, P. Simeone, A. Sacchetti, V. Relli, D. A. A, R. La Sorda, R. Lattanzio, M. Piantelli and S. Alberti (2016). "Trop-2 induces tumor growth through Akt and determines sensitivity to Akt inhibitors." Clin Cancer Res 22(16): 4197-4205. Guncar, G., G. Pungercic, I. Klemencic, V. Turk and D. Turk (1999). "Crystal structure of MHC class II-associated p41 li fragment bound to cathepsin L reveals the structural basis for differentiation between cathepsins L and S." EMBO J. 18: 793-803.
Ikeda, M., M. Yamaguchi, K. Kato, K. Nakamura, S. Shiina, T. Ichikawa-Ando, H. Misaka, K. Myojo, Y. Sugimoto and H. Hamada (2015). "Pr1E11, a novel anti-TROP-2 antibody isolated by adenovirus-based antibody screening, recognizes a unique epitope." Biochem Biophys Res Commun 458(4): 877-882.
Johnson, B. E. and P. A. Janne (2006). "Rationale for a phase II trial of pertuzumab, a HER-2 dimerization inhibitor, in patients with non-small cell lung cancer." Clin Cancer Res 12(14 Pt 2): 4436s-4440s.
Karagiannis GS, Condeelis JS, Oktay MH. Chemotherapy-induced metastasis: mechanisms and translational opportunities. Clin Exp Metastasis. 2018 Apr;35(4):269-284.
Kim H, Guerra E, Baek E, Jeong Y, You H, Yu B, Jang T, Saverio A, Chung C-W, Park C. (2022) LCB84, a TROP2-targeted ADC, for treatment of solid tumors that express TROP-2 using the hu2G10 tumor-selective anti-TROP2 monoclonal antibody, a proprietary site-directed conjugation technology and plasma-stable tumor-selective linker chemistry Cancer Res 82(12_Suppl):Abstract nr 328.
Linnenbach, A. J., B. A. Seng, S. Wu, S. Robbins, M. Scollon, J. J. Pyre, T. Druck and K. Huebner (1993). "Retroposition in a family of carcinoma-associated antigen genes." Mol Cell Biol 13(3): 1507-1515.
Monteran L, Ershaid N, Doron H, Zait Y, Scharff Y, Ben-Yosef S, Avivi C, Barshack I, Sonnenblick A, Erez N. Chemotherapy-induced complement signaling modulates immunosuppression and metastatic relapse in breast cancer. Nat Commun. 2022 Oct 2;13(1):5797.
Mori, Y., K. Akita, K. Ojima, S. Iwamoto, T. Yamashita, E. Morii and H. Nakada (2019). "Trophoblast cell surface antigen 2 (Trop-2) phosphorylation by protein kinase C alpha/delta (PKCalpha/delta) enhances cell motility." J Biol Chem 294(30): 11513-11524.
Nadezhdin, K. D., I. García-Carpio, S. A. Goncharuk, K. S. Mineev, A. S. Arseniev and M. Vilar (2016). "Structural Basis of p75 Transmembrane Domain Dimerization." J Biol Chem 291(23): 12346-12357.
Orsulic, S., Y. Li, R. A. Soslow, L. A. Vitale-Crosss, J. S. Gutkind and H. E. Varmus (2002). "Induction of ovarian cancer by defined multiple genetic changes in a mouse model system." Cancer Cell 1(1): 53-62.
Pavšič, M. (2021). "Trop2 Forms a Stable Dimer with Significant Structural Differences within the Membrane-Distal Region as Compared to EpCAM." International Journal of Molecular Sciences 22(19).
Pavsic, M., G. Guncar, K. Djinovic-Carugo and B. Lenarcic (2014). "Crystal structure and its bearing towards an understanding of key biological functions of EpCAM." Nature Communications 5.
Relli, V., M. Trerotola, E. Guerra and S. Alberti (2018). "Distinct lung cancer subtypes associate to distinct drivers of tumor progression." Oncotarget 9(85): 35528-35540.
Rossi, C., A. Di Lena, R. La Sorda, R. Lattanzio, L. Antolini, C. Patassini, M. Piantelli and S. Alberti (2008). "Intestinal tumour chemoprevention with the antioxidant lipoic acid stimulates the growth of breast cancer." Eur J Cancer 44: 2696 -2704.
Shaked Y. The pro-tumorigenic host response to cancer therapies. Nat Rev Cancer. 2019 Dec;19(12):667-685.
Stoyanova, T., A. S. Goldstein, H. Cai, J. M. Drake, J. Huang and O. N. Witte (2012). "Regulated proteolysis of Trop2 drives epithelial hyperplasia and stem cell self-renewal via beta-catenin signaling." Genes Dev 26(20): 2271-2285.
Trerotola, M., P. Cantanelli, E. Guerra, R. Tripaldi, A. L. Aloisi, V. Bonasera, R. Lattanzio, R. de Lange, U. H. Weidle, M. Piantelli and S. Alberti (2013a). "Up-regulation of Trop-2 quantitatively stimulates human cancer growth." Oncogene 32 222-233.
Trerotola, M., E. Guerra and S. Alberti (2010). "Letter to the editor: efficacy and safety of anti-Trop antibodies." Biochim Biophys Acta 1805(2): 119-120.
Trerotola, M., E. Guerra, Z. Ali, A. L. Aloisi, M. Ceci, P. Simeone, A. Acciarito, P. Zanna, G. Vacca, A. D'Amore, K. Boujnah, V. Garbo, A. Moschella, R. Lattanzio and S. Alberti (2021). "Trop-2 cleavage by ADAM10 is an activator switch for cancer growth and metastasis." Neoplasia 23(4): 415-428.
Trerotola, M., D. Jernigan, Q. Liu, J. Siddiqui, A. Fatatis and L. Languino (2013b). "Trop-2 promotes prostate cancer metastasis by modulating β1 integrin functions." Cancer Res. 73(10): 3155-3167.
Truong, A. H. L., N. Feng, D. Sayegh, B. C. Mak, K. O'Reilly, S. W. Fung, N. Ceric, S. H. Hahn, D. Pereira and H. Findlay (2007). "AR47A6.4.2, a functional naked monoclonal antibody targeting Trop-2, demonstrates in vivo efficacy in human pancreatic, colon, breast and prostate cancer models." Molecular Cancer Therapeutics 6(12): 3334s-3334s.
Voorwerk L, Slagter M, Horlings HM, Sikorska K, van de Vijver KK, de Maaker M, Nederlof I, Kluin RJC, Warren S, Ong S, Wiersma TG, Russell NS, Lalezari F, Schouten PC, Bakker NAM, Ketelaars SLC, Peters D, Lange CAH, van Werkhoven E, van Tinteren H, Mandjes IAM, Kemper I, Onderwater S, Chalabi M, Wilgenhof S, Haanen JBAG, Salgado R, de Visser KE, Sonke GS, Wessels LFA, Linn SC, Schumacher TN, Blank CU, Kok M. Immune induction strategies in metastatic triple-negative breast cancer to enhance the sensitivity to PD-1 blockade: the TONIC trial. Nat Med. 2019 Jun;25(6):920-928.
Zanna, P., M. Trerotola, G. Vacca, V. Bonasera, B. Palombo, E. Guerra, C. Rossi, R. Lattanzio, M. Piantelli and S. Alberti (2007). "Trop-1 Are Conserved Growth Stimulatory Molecules That Mark Early Stages of Tumor Progression." Cancer 110(2): 452-464.

## Claims

1. A composition comprising the 4F6 anti-Trop-2 antibody and/or its conjugates for use in the treatment of a Trop-2-expressing tumors in a subject, wherein said subject is immunosuppressed by anti-tumor treatment, including chemotherapy and radiation.

2. The composition for use according to claim 1, wherein said treatment is performed before tumor removal.

3. The composition for use according to claim 1, wherein said treatment is performed after tumor removal.

4. The composition for use according to claim 1, wherein said treatment is performed in combination with other therapeutic interventions.

5. The composition for use according to claim 4, wherein said additional therapeutic interventions is the administration of an antibody-drug conjugate.

6. The composition for use according to any one of the claims 1-5, wherein said 4F6 anti-Trop-2 antibody conjugates are radioactive isotope-bound conjugates, such as β-particle emitters or α-particle emitters.

7. The composition for use according to one of the claims 1-6, wherein said tumor is selected in the group comprising endometrium, breast, colon-rectum, stomach, lung, ovary, prostate, pancreas, head and neck, kidney, bladder and cervix tumors.

8. 4F6 anti-Trop-2 antibodies conjugated to positron emitters for use in the imaging of metastatic immunosuppressed patients by positron-emission tomography or are conjugated to fluorescent tracers to detect Trop-2-expressing tumors and metastases during surgery.
